# EUROPEAN PATENT SPECIFICATION

(11) **EP 3 856 767 B1**
(45) Date of publication and mention of the grant of the patent: **27.03.2024**
(21) Application number: 19779866.3
(22) Date of filing: 27.09.2019
(51) Int. Cl.: C07K 14/605, A61K 38/26, A61K 38/00

(54) **FORMULATIONS OF GLUCAGON-LIKE-PEPTIDE-2 (GLP-2) ANALOGUES**
FORMULIERUNGEN VON ANALOGA DES GLUCAGON-LIKE-PEPTID-2 (GLP-2)
FORMULATIONS D'ANALOGUES GLUCAGON-LIKE-PEPTIDE-2 (GLP-2)

(30) Priority: 28.09.2018 EP 18197750
(43) Date of publication of application: 04.08.2021
(73) Proprietor: Zealand Pharma A/S, 2860 Søborg (DK)
(72) Inventor: PARSHAD, Henrik Rajesh Kumar, 2860 Søborg (DK); GIEHM, Lise, 2860 Søborg (DK); MELANDER, Claes, 2860 Søborg (DK)
(74) Representative: Mewburn Ellis LLP
(86) International application number: PCT/EP2019/076304
(87) International publication number: WO 2020/065063

(56) References cited:
- WO-A1-2004/105790
- WO-A1-2018/142363
- WO-A2-2005/049061
- WO-A2-2006/117565
- US-A1- 2002 025 933
- KAROLINA L. ZAPADKA ET AL: "Factors affecting the physical stability (aggregation) of peptide therapeutics", INTERFACE FOCUS, vol. 7, no. 6, 20 October 2017 (2017-10-20), page 20170030, XP055598856, GB ISSN: 2042-8898, DOI: 10.1098/rsfs.2017.0030

## Description

### Field of the Invention

The present invention relates to formulations of glucagon-like-peptide-2 (GLP-2) analogues and their medical use, for example in the treatment and/or prevention of stomach and/or bowel-related disorders and for ameliorating side effects of chemotherapy and radiation therapy.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH. It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not a useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day.

US 5,789,379 discloses GLP-2 analogues for administration by injection. The analogues were provided as powdered peptides and mixed with phosphate buffered saline (PBS) prior to injection at pH of 7.3-7.4 with a GLP-2 concentration of 130 mg/mL. In some instances, the GLP-2/PBS composition was mixed with gelatin to provide a depot formed from a solution of 130 mg/l GLP-2 in PBS/15% gelatin. US 5,789,379 does not disclose stable aqueous liquid formulations of GLP-2 analogues and the GLP-2 analogues are generally reconstituted from powder prior to injection.

In WO 97/39031 and US 6,184,201, the GLP-2 analogue, [Gly²]GLP-2 is disclosed. Here the alanine in position 2 has been replaced with glycine to make the peptide resistant to DPP IV cleavage. As with US 5,789,379, the GLP-2 analogue was provided as a powdered peptide and mixed with saline, PBS or 5% dextrose prior to injection, optionally adding acetic acid as a solubility enhancer.

WO 02/066511 describes GLP-2 analogues having an extended half-life *in vivo* and their use as medicaments in the treatment of gastrointestinal disorders, such as inflammatory bowel diseases. The GLP-2 analogues were stored in lyophilized form and reconstituted for administration in media, for example using saline or PBS.

WO 01/41779 describes the use of h[Gly²]GLP-2 as a pre-treatment for inhibiting chemotherapy induced apoptosis and promoting cell survival. The h[Gly²]GLP-2 is delivered by subcutaneous or intravenous injection or infusion after reconstituting the analogue in PBS.

WO 2001/049314 is directed to formulations of GLP-2 peptides and analogues thereof exhibiting superior stability following storage and/or exposure to elevated temperatures. The GLP-2 compositions comprise a GLP-2 peptide or an analogue thereof, a phosphate buffer, L-histidine, and mannitol.

WO 2006/117565 describes GLP-2 analogues which comprise one of more substitutions as compared to [hGly²]GLP-2 and which improved biological activity *in vivo* and/or improved chemical stability, e.g. as assessed in *in vitro* stability assays. In particular, GLP-2 analogues are described which have substitutions at one or more of positions 8, 16, 24 and/or 28 of the wild-type GLP-2 sequence, optionally in combination with further substitutions at position 2 and one or more of positions 3, 5, 7, 10 and 11, and/or a deletion of one or more of amino acids 31 to 33. These substitutions may also be combined with the addition of a N-terminal or C-terminal stabilizing peptide sequence. The daily or twice daily administration of these GLP-2 analogues is also described. Among the molecules disclosed in WO 2006/117565 is glepaglutide (ZP1848) which has been designed to be stable in liquid formulations, and is typically administered by daily dosing using an injection pen.

WO 2005/049061 relates to formulations of GLP-1 agonists that include propylene glycol to reduce clogging of devices used to administer the peptide and for reducing deposits of the peptide on production equipment.

It remains a problem in this area to improve the formulation of GLP-2 analogues, in particular to provide stable liquid formulations that are capable of long term storage without undue levels of physical degradation of the active monomeric form of the peptide occurring. The stability of formulations of GLP-2 analogues is a particular problem in situations where it is desirable to include a preservative in the formulation, either for regulatory reasons and/or where the formulation is a multi-dose formulation, as preservatives tend to destabilise formulations of peptide drugs in an unpredictable manner.

### Summary of the Invention

Broadly, the present invention is based on studies reported in the examples that led to surprising findings relating to liquid formulations of GLP-2 analogues comprising a preservative can be stabilised for long term storage as liquids and/or as multi-dose liquid formulations. Broadly, the present inventors found that reducing the pH of the formulation to below 7.0, e.g. to a pH between about pH 5.0 and about 6.8, was able to offset the effect that the addition of a preservative such as meta-cresol has on the physical stability of the formulation. Alternatively or additionally, the present inventors found that the addition of an excipient such as propylene glycol was similarly able to offset the effect that the addition of a preservative, such as meta-cresol, has on the physical stability of the formulation. This opens up the possibility of being able to deliver the GLP-2 analogues as multi-dose formulations. This further opens up the possibility of providing the formulations of the GLP-2 analogue in the form of a drug delivery device, and particularly multi-dose delivery devices, such as a pre-filled syringe, an adjustable dose auto-injector, a disposable auto-injector, a wearable injector or an infusion pump, thereby providing patients with a ready-to-use formulation in a simpler, safer and more patient-friendly device.

Furthermore, the present inventors found that the inclusion of the preservative in the GLP-2 analogue formulations did not have any significant adverse effect on the chemical stability of the peptide. Thus, the components of the formulation and their amounts provide a formulation with at least 90% content of the GLP-2 analogue and with less than 10% of chemical degradation products at storage for at least 18 months at 2-8°C.

Accordingly, in a first aspect, the present invention provides a stable liquid pharmaceutical multi-dose formulation, the formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
   (a) the GLP-2 analogue at a concentration of about 1 mg/mL to about 30 mg/mL;
   (b) meta-cresol or phenol as a preservative at a concentration of 1.0 to 5.0 mg/mL;
   (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
   (d) a non-ionic tonicity modifier selected from the group consisting of mannitol, sucrose, glycerol, sorbitol and trehalose at a concentration of about 20 mM to about 360 mM;
   (e) a pH of about 5.0 to about 6.8.

In a further aspect, the present invention provides a stable liquid pharmaceutical multi-dose formulation, the formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
   (a) the GLP-2 analogue at a concentration of about 1 mg/mL to about 30 mg/mL;
   (b) meta-cresol or phenol as a preservative at a concentration of 1.0 to 5.0 mg/mL;
   (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
   (d) a non-ionic tonicity modifier selected from the group consisting of mannitol, sucrose, glycerol, sorbitol and trehalose at a concentration of about 20 mM to about 360 mM;
   (e) a pH of about 5.5 to about 7.0; and
   (f) propylene glycol at a concentration of 20 mM to 300 mM.

In some embodiments, the formulations of the present invention comprises propylene glycol at a concentration of 50 mM to 300 mM, more preferably at a concentration of 50 mM to 200 mM, more preferably at a concentration of 50 mM to 150 mM, more preferably at a concentration of 150 mM to 200 mM.

In a further aspect, the present invention provides an article of manufacture or a kit comprising a container holding the stable pharmaceutical formulation of the present invention.

In a further aspect, the present invention provides a delivery device containing a liquid formulation comprising a GLP-2 analogue of present invention.

In a further aspect, the present invention provides a formulation of the glucagon-like peptide 2 (GLP-2) analogue of the present invention for use in therapy.

In a further aspect, the present invention provides a formulation of the glucagon-like peptide 2 (GLP-2) analogue of the present invention for use in a method for the treatment and/or prevention of a stomach and/or bowel-related disorder in a human patient.

In a further aspect, the present invention provides a process for producing a stable liquid multi-dose pharmaceutical formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
wherein the process comprising formulating (a) the GLP-2 analogue at a concentration of about 1 mg/mL to about 30 mg/mL; (b) meta-cresol or phenol as a preservative at a concentration of 1.0 to 5.0 mg/mL; (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (d) a non-ionic tonicity modifier selected from the group consisting of mannitol, sucrose, glycerol, sorbitol and trehalose at a concentration of about 20 mM to about 360 mM; at a pH of about 5.0 to about 6.8.

In a further aspect, the present invention provides a process for producing a stable liquid multi-dose pharmaceutical formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
wherein the process comprising formulating (a) the GLP-2 analogue at a concentration of about 1 mg/mL to about 30 mg/mL; (b) meta-cresol or phenol as a preservative at a concentration of 1.0 to 5.0 mg/mL; (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (d) a non-ionic tonicity modifier selected from the group consisting of mannitol, sucrose, glycerol, sorbitol and trehalose at a concentration of about 20 mM to about 360 mM; (e) propylene glycol at a concentration of 20 mM to 300 mM; at a pH of about 5.5 to about 7.0.

In a further aspect, the present invention provides the use of a formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
for providing a multi-dose liquid pharmaceutical formulation which is stable for 18 months when stored at 2-8°C, wherein the formulation comprises:
   (a) the GLP-2 analogue at a concentration of about 1 mg/mL to about 30 mg/mL;
   (b) meta-cresol or phenol as a preservative at a concentration of 1.0 to 5.0 mg/mL;
   (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
   (d) a non-ionic tonicity modifier selected from the group consisting of mannitol, sucrose, glycerol, sorbitol and trehalose at a concentration of about 20 mM to about 360 mM;
   (e) a pH of about 5.0 to about 6.8.

In a further aspect, the present invention provides the use of a formulation comprising a glucagon-like peptide 2 (GLP-2) analogue, wherein the GLP-2 analogue is represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
for providing a multi-dose liquid pharmaceutical formulation which is stable for 18 months when stored at 2-8°C, wherein the formulation comprises:
   wherein the formulation comprises:
   (a) the GLP-2 analogue at a concentration of about 1 mg/mL to about 30 mg/mL;
   (b) meta-cresol or phenol as a preservative at a concentration of 1.0 to 5.0 mg/mL;
   (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
   (d) a non-ionic tonicity modifier selected from the group consisting of mannitol, sucrose, glycerol, sorbitol and trehalose at a concentration of about 20 mM to about 360 mM;
   (e) a pH of about 5.5 to about 7.0; and
   (f) propylene glycol at a concentration of 20 mM to 300 mM.

In a further aspect, the present invention relates to a stable liquid multi-dose pharmaceutical formulation, the formulation comprising a glucagon-like peptide 2 (GLP-2) analogue, wherein the GLP-2 analogue is represented by the formula:

R¹- His-Gly-Glu-Gly-X5-Phe-Ser-Ser-Glu-Leu-X11-Thr-Ile-Leu-Asp-Ala-Leu-Ala-Ala- Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z² is a peptide sequence of 6 amino acid units of Lys;
or a pharmaceutically acceptable salt or derivative thereof.

In all of the aspects of the invention described herein, the buffer may a histidine buffer.

In all of the aspects of the invention described herein, the non-ionic tonicity modifier may be selected from the group consisting of mannitol, sucrose, glycerol and sorbitol.

In this aspect of the present invention, the formulations comprising the glucagon-like peptide 2 (GLP-2) analogue may be used for the treatment and/or prevention of stomach and/or bowel-related disorders such as ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS). Alternatively or additionally, the glucagon-like peptide 2 (GLP-2) analogue may be used for the treatment and/or prevention of stomach and/or bowel-related disorders such radiation enteritis, infectious or post-infectious enteritis, or small intestinal damage due to toxic or other chemotherapeutic agents. In this case, treatment with the GLP-2 analogue may optionally be combined with one or more anti-cancer therapies, and may therefore comprise administering one or more chemotherapeutic agent(s) to the patient or treating the patient with radiation therapy.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂(SEQ ID NO: 5); or
ZP2530 H-HGEGTFSSELATILDALAARDFIAWLIATKITD-OH (SEQ ID NO: 6).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂₍SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

Embodiments of the present invention will now be described by way of example and not limitation. However, various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Detailed Description of the Invention

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present.

Preferred compounds of the present invention have at least one GLP-2 biological activity, in particular in causing growth of the intestine. This can be assessed in *in vivo* assays, for example as described in the examples of (e.g.) WO 2006/117565, in which the mass of the intestine, or a portion thereof is determined after a test animal has been treated or exposed to a GLP-2 analogue.

The liquid formulations according to the present invention are preferably an isosmotic liquid formulation. "Isosmotic" means that the formulations of the present invention have the same or a similar osmotic pressure with bodily fluids. Preferably, the formulations of the present invention have an osmolality of about 300 ± 60 mOsm as measured by an osmometer.

The formulations of the present invention generally contain the GLP-2 analogue at a concentration of about 1 mg/mL to about 30 mg/mL, more preferably at a concentration of about 1 mg/mL to about 20 mg/mL, more preferably at a concentration of about 1 mg/mL to about 15 mg/mL, and more preferably at a concentration of about 2 mg/mL to about 10 mg/mL, and most preferably at a concentration of about 2 mg/mL to about 20 mg/mL. In further embodiments, the present invention generally contain the GLP-2 analogue at a concentration of about 2 mg/mL, 5 mg/mL, 10 mg/mL or 20 mg/mL. The amount of GLP-2 analogue and its degradation products may be determined by HPLC using techniques well known to the skilled person.

In some embodiments, the formulation of the present invention may be used in a once or twice daily dosage regime. In some cases, the formulation of the present invention may be used in a once or twice weekly dosage regime. Alternatively or additionally, the dosing regime of the GLP-2 analogues of the present invention may comprise a plurality or course of doses separated in time by 2 days, 2.5 days, 3 days, 3.5 days, 4 days, 5 days, 6 days, 7 days, 8 days, 9 days, 10 days, 11 days or 12 days. In a preferred embodiment, the doses are separated in time by 3 days, 3.5 days, 4 days, 5 days, 6 days, 7 days or 8 days. In a preferred embodiment, doses are separated in time by 3 days, 3.5 days, 4 days or 7 days. As will be appreciated in the art, the time between doses may be varied to some extent so that each and every doses is not separated by precisely the same time. This will often be directed under the discretion of the physician. Thus, doses may be separated in time by a clinically acceptable range of times, e.g. from about 2 days to about 10 days, or from about 3 or 4 days to about 7 or 8 days.

The formulations of the present invention are stable liquid pharmaceutical formulations of GLP-2 analogues. A "stable" formulation is one in which the peptide therein essentially retains its physical stability and/or chemical stability and/or biological activity upon storage. Preferably, the formulation essentially retains its physical and chemical stability, as well as its biological activity upon storage. The storage period is generally selected based on the intended shelf-life of the formulation. The formulations of the present invention are provided as stable liquid formulations, e.g. stable aqueous liquid formulations. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example. In the present invention, "stable" formulations include formulations in which at least 80%, more preferably at least 90%, more preferably at least 95%, more preferably at least 96%, more preferably at least 97%, more preferably at least 98%, and most preferably at least 99% of the GLP-2 analogue is active in the formulation after it has been stored at 2-8°C for at least 18 months.

Stability can be measured at a selected temperature for a selected time period, for example using elevated temperature to reduce the period over which a formulation is tested. Generally, storage at a temperature between 2 to 8°C denotes storage under normal refrigerated conditions. In certain embodiments, the formulation is stable under such conditions for at least 12 months, more preferably at least 18 months, more preferably at least 24 months. Stability can be evaluated qualitatively and/or quantitatively in a variety of different ways, including evaluation of degradation products (e.g. using HPLC and/or LC-MS), aggregate formation (e.g. using size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, etc.

A peptide "retains its physical stability" in a pharmaceutical formulation if it shows no sign (or very little sign) of aggregation, precipitation and/or denaturation upon e.g. visual examination of colour and/or clarity, or as measured by UV light scattering, dynamic light scattering, circular dichroism, or by size exclusion chromatography and is considered to still retain its biological activity.

A peptide "retains its chemical stability" in a pharmaceutical formulation, if the chemical stability at a given time is such that the peptide is considered to still retain its biological activity as defined below. Chemical stability can be assessed by detecting and quantifying chemically altered forms of the peptide. Chemical alteration may involve isomerization, oxidation, size modification (e.g. clipping) which can be evaluated using HPLC or size exclusion chromatography, SDS-PAGE and/or mass spectrometry, for example. Other types of chemical alteration include charge alteration n (e.g. occurring as a result of deamidation) which can be evaluated by HPLC or ion-exchange chromatography or icIEF, for example.

### GLP-2 analogues

The GLP-2 analogues present in the formulations of the present invention have one or more amino acid substitutions, deletions, inversions, or additions compared with native GLP-2 and as defined above. This definition also includes the synonym terms GLP-2 mimetics and/or GLP-2 agonists. Further, the analogue of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C₁-C₄ alkyl. Furthermore, one or more side groups, or terminal groups, may be protected by protective groups known to the ordinarily-skilled peptide chemist. The α-carbon of an amino acid may be mono- or di-methylated.

In some aspects, the liquid formulations of the present invention employ a glucagon-like peptide 2 (GLP-2) analogue represented by the formula:

R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In an embodiment of the present invention, the glucagon-like peptide 2 (GLP-2) analogue is ZP1848 H-HGEGTFSSELATILDALAARDFIAWLIATKITDKKKKKK-NH₂ (SEQ ID NO: 1).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂₍SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

In an embodiment of the present invention, the glucagon-like peptide 2 (GLP-2) analogue is ZP1846 H-HGEGSFSSELSTILDALAARDFIAWLIATKITDKKKKKK-NH_{2 (}SEQ ID NO: 7).

It should be understood that the peptides (drug substance) of the invention might also be provided in the form of a salt or other derivative. Salts include pharmaceutically acceptable salts, such as acid addition salts and basic salts. Examples of acid addition salts include hydrochloride salts, citrate salts, chloride salts and acetate salts. Preferably, the salt is acetate. Examples of basic salts include salts where the cation is selected from alkali metals, such as sodium and potassium, alkaline earth metals, such as calcium, and ammonium ions ⁺N (R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences", 17th edition. Ed. Alfonso R. Gennaro (Ed.), Mark Publishing Company, Easton, PA, U.S.A., 1985 and more recent editions, and in the Encyclopaedia of Pharmaceutical Technology.

In preferred embodiments, the acetate salt of a GLP-2 analogue of the invention is selected from the group consisting of ZP1848-acetate, ZP2949-acetate, ZP2711-acetate, ZP2469-acetate, ZP1857-acetate, ZP2530-acetate, ZP1846-acetate, ZP1855-acetate and ZP2242-acetate. In the present context, the term "ZP1848-acetate" refers to the ZP1848 molecule is in the form of an acetate salt. The acetate salts of GLP-2 analogues may be represented by the formula (GLP-2 analogue), x(CH₃COOH) where x is 1.0 to 8.0, i.e. where x is 1.0, 2.0, 3.0, 4.0, 5.0, 6.0, 7.0 or 8.0. In any composition of the acetate salts of the GLP-2 analogues, there may be molecules with different number of acetate molecules so that x is not necessarily a whole integer. In some cases, x is from 4.0 to 8.0, x is from 6.0 to 8.0, or x is from 4.0 to 6.5. In some cases is from x is from 4.0 to 6.0, x is from 2.0 to 7.0, x is from 3.0 to 6.0, x is from 4.0 to 6.0 or x is 4.0 to 8.0.

In a preferred embodiment, the GLP-2 analogue is ZP1848-acetate or H-HGEGTFSSELATILDALAARDFIAWLIATKITDKKKKKK-NH₂ acetate (SEQ ID NO: 1), (H-HGEGTFSSELATILDALAARDFIAWLIATKITDKKKKKK-NH2), x(CH₃COOH) where x is 1.0 to 8.0.

Accordingly, in a further aspect, the present invention provides solid compositions comprising an acetate salt of a glucagon-like peptide 2 (GLP-2) analogue. The solid compositions are useful for formulating with the excipients used to make the liquid formulations of the present invention. In one embodiment, the present invention provides a solid composition comprising an acetate salt of a glucagon-like peptide 2 (GLP-2) analogue having the formula:
(H-HGEGTFSSELATILDALAARDFIAWLIATKITDKKKKKK-NH2), x(CH₃COOH) where x is 1.0 to 8.0.

An upper limit of 8.0 acetate molecules per GLP-2 analogue equates to an acetate content of less than 11% acetate and may be formulated to have a viscosity between 0.8 and 2.0 mPa/sec measured at 25°C.

The range of the number of acetate molecules associated with each molecule of the GLP-2 analogues defines a molecular weight range for this component of the formulation. For example, for the acetate salts of ZP1848, the range of the number of acetate molecules associated with each molecule of the GLP-2 analogues defines a molecular weight range of the ZP1848-acetate. By way of example, 1 acetate equivalent with each molecule of ZP1848 provides a molecular weight = 4316 + 60 = 4376 Da. Accordingly, the molecular weights for increasing acetate equivalents with ZP1848 are as follows: 1 acetate equivalent = 4376 Da; 2 acetate equivalents = 4436 Da; 3 acetate equivalents = 4496 Da; 4 acetate equivalents =4556 Da; 5 acetate equivalents = 4616 Da; 6 acetate equivalents = 4676 Da; 7 acetate equivalents = 4736 Da and 8 acetate equivalents = 4796 Da. This in turn defines molecular weight ranges as follows: 1-8 acetate equivalents = 4376 Da - 4796 Da; 4-8 acetate equivalents = 4556 Da - 4796 Da and 6-8 acetate equivalents = 4676 Da - 4796 Da.

Other derivatives of the GLP-2 analogues of the invention include coordination complexes with metal ions such as Mn²⁺ and Zn²⁺, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

Z¹ and Z² are independently present and/or absent or a peptide sequence of 1-6 amino acid units of Lys, i.e. 1, 2, 3, 4, 5 or 6 Lys residues. The Lys residues may have either D- or L-configuration, but have an L-configuration. Particularly preferred sequences Z are sequences of four, five or six consecutive lysine residues, and particularly six consecutive lysine residues. Exemplary sequences Z are shown in WO 01/04156. In certain embodiments, Z¹ is absent. In such cases, Z² may be either present or absent.

### Formulations of the GLP-2 analogues

The formulation of the GLP-2 analogues is a ready-to-use formulation. The term "ready-to-use" as used herein, refers to a formulation that does not require constitution or dilution with a prescribed amount of diluent, e.g., water for injection or other suitable diluent, before use by the designated route of administration.

As described herein, the liquid formulations of the GLP-2 analogues of the present invention include a buffer, a non-ionic tonicity modifier and arginine q.s. to provide the pH of the final formulation. In accordance with normal pharmaceutical practice, the formulations of the present invention are sterile and/or free from reducing agent. In some cases, the liquid formulations of the present invention are aqueous, liquid formulations.

The term "buffer" as used herein denotes a pharmaceutically acceptable excipient which stabilizes the pH of a pharmaceutical formulation. Suitable buffers are well known in the art and can be found in the literature. By way of example, the buffer may be selected from the group consisting of a histidine buffer, mesylate buffer, acetate buffer, glycine buffer, lysine buffer, TRIS buffer, Bis-Tris buffer and MOPS buffer. In preferred embodiments, the buffer is selected from the group consisting of a histidine buffer, acetate buffer and mesylate buffer as these buffers provided stable formulations in which the GLP-2 analogues dissolved and did not become viscous, cloudy or precipitate the peptide drug. In preferred embodiments, the buffer is a histidine buffer, e.g. L-histidine. Generally, the buffer will be present at a concentration of about 5 mM to about 50 mM, more preferably at a concentration of about 5 mM to about 25 mM, and most preferably at a concentration of about 15 mM. Preferably, the buffer is not a phosphate buffer, a citrate buffer, citrate/Tris buffer and/or succinate buffer.

The term "tonicity modifier" as used herein denotes pharmaceutically acceptable tonicity agents that are used to modulate the tonicity of the formulation. The formulations of the present invention are preferably isosmotic, that is they have an osmotic pressure that is substantially the same as human blood serum. The tonicity modifiers used in the formulations are preferably non-ionic tonicity modifiers and are preferably selected from the group consisting of mannitol, sucrose, glycerol, sorbitol and trehalose. More preferably, the non-ionic tonicity modifier is selected from the group consisting of mannitol, sucrose, glycerol and sorbitol A preferred non-ionic tonicity modified is mannitol, e.g. D-mannitol. The concentration of the tonicity modifier will be dependent on the concentration of other components of the formulation, especially where the formulation is intended to be isosmotic. Typically, the non-ionic tonicity modifier will be employed at a concentration of about 90 mM to about 360 mM, more preferably at a concentration of about 150 mM to about 250 mM, and most preferably at a concentration of about 230 mM.

Generally, the components and amounts of the liquid formulations of the present invention are chosen to provide a formulation with a pH of about 5.0 to about 6.8, in aspects of the present invention where a reduction in pH is used to compensate for the addition of preservative to the formulation. In an preferred embodiment, where a reduction in pH is used to compensate for the addition of preservative to the formulation, the formulations may have a pH between about 6.2 and about 6.8. Alternatively or additionally, in aspects of the present invention in which propylene glycol is present the formulations may have a pH between about 5.5 and about 7.0, such as about 6.0 and about 7.0, about 6.8 and about 7.0.

In a further embodiment, the liquid formulations of the present invention comprises:
(a) the GLP-2 analogue at a concentration of about 2 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM;
(e) a pH of about 5.5 to about 7.0; and
(f) propylene glycol at a concentration of 20 mM to 300 mM.

In a further embodiment, the liquid formulations of the present invention comprises:
(a) the GLP-2 analogue at a concentration of about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 50 mM to about 230 mM; and
(e) a pH of about 5.0 to about 7.0; and
(f) propylene glycol at a concentration of 50 mM to 200 mM.

In a further embodiment, the liquid formulations of the present invention consists of the GLP-2 analogue at a concentration of about 10 mg/mL; meta-cresol or phenol as a preservative at a concentration of 3.0 to 4.0 mg/mL; a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 10 mM to about 20 mM; ) a non-ionic tonicity modifier selected from the group consisting of mannitol, sucrose, glycerol, sorbitol and trehalose at a concentration of about 50 mM to about 230 mM; and propylene glycol at a concentration of 50 mM to 200 mM, the formulation having a pH of about 5.5 to about 7.0.

In a further embodiment, the liquid formulations of the present invention consists of the GLP-2 analogue at a concentration of about 10 mg/mL; meta-cresol as a preservative at a concentration of 3.0 to 4.0 mg/mL; histidine buffer at a concentration of about 10 mM to about 20 mM; mannitol as a non-ionic tonicity modifier at a concentration of about 50 mM to about 230 mM; and propylene glycol at a concentration of 50 mM to 200 mM, the formulation having a pH of about 5.5 to about 7.0.

In a further embodiment, the liquid formulations of the present invention comprises:
(a) the GLP-2 analogue at a concentration of about 1 mg/mL to about 15 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM; and
(e) has a pH of about 6.0 to about 6.8.

In a further embodiment, the liquid formulations of the present invention comprises:
(a) the GLP-2 analogue at a concentration of about 2 mg/mL or about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 200 mM to about 250 mM; and
(e) has a pH of about 6.0 to about 6.8.

In a further embodiment, the liquid formulations of the present invention comprises the GLP-2 analogue at a concentration of about 2 mg/mL, about 10 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

In a further embodiment, the liquid formulations of the present invention comprises the GLP-2 analogue at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention comprises ZP1848-acetate or H-HGEGTFSSELATILDALAARDFIAWLIATKITDKKKKKK-NH2 acetate (SEQ ID NO: 1). at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention comprises ZP1846 H-HGEGSFSSELSTILDALAARDFIAWLIATKITDKKKKKK-NH2 (SEQ ID NO: 7); at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of the GLP-2 analogue at a concentration of about 2 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

In a further embodiment, the liquid formulations of the present invention consists of the GLP-2 analogue at a concentration of about 2 mg/mL, about10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of ZP1848-acetate or H-HGEGTFSSELATILDALAARDFIAWLIATKITDKKKKKK-NH2 acetate (SEQ ID NO: 1). at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of ZP1846 H-HGEGSFSSELSTILDALAARDFIAWLIATKITDKKKKKK-NH2 (SEQ ID NO: 7); at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulation is selected from the group consisting of an aqueous liquid formulation, a liquid formulation in various hydrophilic or hydrophobic solvents, an emulsion and a liquid suspension. In a preferred embodiment, the liquid formulation is an aqueous liquid formulation. In some cases, the liquid formulations of the present invention are non-aqueous, liquid formulations.

Preferably, the glucagon-like peptide 2 (GLP-2) analogue are administered to patients parenterally, preferably by injection, most typically by subcutaneous injection, intramuscular injection, intravenous injection or intraperitoneal injection. Administration by subcutaneous injection is preferred. The injection may be carried out by a physician, nurse or other healthcare professional, or may be self-administered by the patient. As set out herein, in some aspects, the formulations of the present invention have a viscosity that facilitates loading of the formulation into a pre-filled syringe, an injection pen or other injector device. This may have the advantage of pre-determining the dose of the formulation administered to the patient, e.g. without the need for measurement from a multi-dose vial. Accordingly, in other aspects, the present invention provides an article of manufacture or a kit comprising a container holding the stable, such as e.g. an aqueous stable pharmaceutical formulation of the GLP-2 analogue according to the present invention or a pre-filled syringe or injector device or injector pen containing an aqueous liquid formulation comprising the GLP-2 analogue according to the present invention.

### Medical Conditions

The GLP-2 analogue formulations of the present invention are useful as a pharmaceutical agent for preventing or treating an individual suffering from gastro-intestinal disorders, including the upper gastrointestinal tract of the oesophagus by administering an effective amount of a GLP-2 analogue, or a salt thereof as described herein. The stomach and intestinal-related disorders include ulcers of any aetiology (e.g., peptic ulcers, drug-induced ulcers, ulcers related to infections or other pathogens), digestion disorders, malabsorption syndromes, short-bowel syndrome, cul-de-sac syndrome, inflammatory bowel disease, celiac sprue (for example arising from gluten induced enteropathy or celiac disease), tropical sprue, hypogammaglobulinemic sprue, enteritis, ulcerative colitis, small intestine damage, and chemotherapy induced diarrhoea/mucositis (CID).

As mentioned above, in general individuals who would benefit from increased small intestinal mass and consequent and/or maintenance of normal small intestine mucosal structure and function are candidates for treatment with the present GLP-2 analogues. Particular conditions that may be treated with GLP-2 analogue include the various forms of sprue including celiac sprue which results from a toxic reaction to alpha-gliadin from heat and may be a result of gluten-induced enteropathy or celiac disease, and is marked by a significant loss of villae of the small bowel; tropical sprue which results from infection and is marked by partial flattening of the villae; hypogammaglobulinemic sprue which is observed commonly in patients with common variable immunodeficiency or hypogammaglobulinemia and is marked by significant decrease in villus height. The therapeutic efficacy of the GLP-2 analogue treatment may be monitored by enteric biopsy to examine the villus morphology, by biochemical assessment of nutrient absorption, by patient weight gain, or by amelioration of the symptoms associated with these conditions.

Another particular condition which may be treated with the GLP-2 analogues of the invention, or for which the GLP-2 analogues may be useful therapeutically and/or prophylactically is short bowl syndrome (SBS), also known as short gut syndrome or simply short gut, which results from surgical resection, congenital defect or disease-associated loss of absorption in the bowel in which patients are subsequently unable to maintain fluid, electrolyte, and nutrient balances on a conventional diet. Despite an adaptation that occurs generally in the two years after resection, SBS patients have reduced dietary uptake and fluid loss.

Other conditions that may be treated with the GLP-2 analogues of the invention, or for which the GLP-2 analogues may be useful prophylactically, include in addition to the above mentioned radiation enteritis, infectious or post-infectious enteritis, and small intestinal damage due to cancer-chemotherapeutic or toxic agents.

The GLP-2 analogues may also be used for the treatment of malnutrition, for example cachexia and anorexia.

A particular embodiment of the invention is concerned with using the present peptides for the prevention and/or treatment of intestinal damage and dysfunction. Such damage and dysfunction is a well-known side effect of cancer-chemotherapy treatment. Chemotherapy administration is frequently associated with unwanted side effects related to the gastronintestinal system such as mucositis, diarrhoea, bacterial translocation, malabsorption, abdominal cramping, gastrointestinal bleeding and vomiting. These side effects are clinical consequences of the structural and functional damage of the intestinal epithelium and frequently make it necessary to decrease the dose and frequency of chemotherapy.

Administration of the present GLP-2 peptide analogues may enhance trophic effect in the intestinal crypts and rapidly provide new cells to replace the damaged intestinal epithelium following chemotherapy. The ultimate goal achieved by administering the present peptides is to reduce the morbidity related to gastrointestinal damage of patients undergoing chemotherapy treatment while creating the most optimal chemotherapy regime for the treatment of cancer. Concomitant prophylactic or therapeutic treatment may be provided in accordance with the present invention to patients undergoing or about to undergo radiation therapy.

The stem cells of the small intestinal mucosa are particularly susceptible to the cytotoxic effects of chemotherapy due to their rapid rate of proliferation (Keefe et al., Gut, 47: 632-7, 2000). Chemotherapy-induced damage to the small intestinal mucosa is clinically often referred to as gastrointestinal mucositis and is characterized by absorptive and barrier impairments of the small intestine. For example, it has been shown that, the broadly used chemotherapeutic agents, 5-FU, irinotecan and methothrexate increase apoptosis leading to villus atrophy and crypt hypoplasia in the small intestine of rodents (Keefe et al., Gut 47: 632-7, 2000; Gibson et al., J Gastroenterol. Hepatol. Sep;18(9):1095-1100, 2003; Tamaki et al., J. Int. Med. Res. 31(1):6-16, 2003). Chemotherapeutic agents have been shown to increase apoptosis in intestinal crypts at 24 hours after administration and subsequently to decrease villus area, crypt length, mitotic count per crypt, and enterocyte height three days after chemotherapy in humans (Keefe et al., Gut, 47: 632-7, 2000). Thus, structural changes within the small intestine directly lead to intestinal dysfunction and in some cases diarrhoea.

Gastrointestinal mucositis after cancer chemotherapy is an increasing problem that is essentially untreatable once established, although it gradually remits. Studies conducted with the commonly used cytostatic cancer drugs 5-FU and irinotecan have demonstrated that effective chemotherapy with these drugs predominantly affects structural integrity and function of the small intestine while the colon is less sensitive and mainly responds with increased mucus formation (Gibson et al., J. Gastroenterol. Hepatol. Sep;18(9):1095-1100, 2003; Tamaki et al., J Int. Med. Res. 31(1):6-16, 2003).

The formulations of the present invention comprising GLP-2 analogues may be useful in the prevention and/or treatment of gastrointestinal injury and side effects of chemotherapeutic agents. This potentially important therapeutic application may apply to currently used chemotherapeutic agents such as but not limited to: 5-FU, Altretamine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Mesna, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, and Vinorelbine.

### Delivery of the Formulations

In some aspects, the present invention relates to a ready-to-use formulation of GLP-2 analogues, intended for parenteral administration, and suitable for use in e.g. vials, pre-filled syringes, infusion pumps, wearable injectors, disposable auto-injectors or adjustable dose auto-injectors.

### Examples

The following examples are provided to illustrate preferred aspects of the invention and are not intended to limit the scope of the invention. The GLP-2 analogues administered according to the dosage regimes described herein can be made according to the methods such as solid phase peptide synthesis described in WO 2006/117565,

### Example 1. Synthesis of ZP1848-acetate and similar GLP-2 analogues

ZP1848-acetate peptide was synthesized using an Fmoc Solid Phase Peptide Synthesis (SPPS) approach with standard coupling conditions. After completed synthesis, the peptide sequence was deprotected and cleaved from the solid support, and the crude peptide was purified using preparative reversed-phase HPLC. The peptide was converted to the desired acetate salt form by applying a mobile phase during the final chromatographic step with an appropriate concentration of acetic acid and subsequent lyophilization. The resulting drug substance product had an acetate content below 11% or below 8 equivalents of acetate: batch 1 (6% acetate, 4.6 equivalents of acetate), batch 2 (7% acetate, 5.4 equivalents of acetate) and batch 3 (6% acetate, 4.6 equivalents of acetate). This synthesis and purification protocol may be adapted for making other GLP-2 analogues used in the formulations of the present invention.

### Example 2: Effect of adding preservative to GLP-2 analogue formulations

Excipient stock solutions of L-histidine (200 mM), mannitol (500 mM), m-cresol (1034 mg/mL)) and propylene glycol (1036 mg/ml) were prepared. Excipient solutions were mixed according to the desired composition (see Table 1) and stock solution of ZP1848 (approx. 40 mg/mL) was added. The volume was adjusted to 90% of final volume, pH was adjusted with acetic acid (200 mM) and/or L-arginine (200 to 250 mM), m-cresol was added, and the volume was adjusted to give the final volume. The formulations were sterile-filtered and filled in pharmaceutically acceptable containers, such as vials or cartridges.

The results of this experiment are shown in Table 1. This example shows that addition of the preservative meta-cresol in formulation 1 reduced the physical stability of the formulation and led to precipitation of the active drug ZP1848. The example also shows that this effect can be circumvented by lowering the pH of the formulation as shown from the results with formulations at pH 6.0 and pH 5.0.

### Example 3: Stable formulations at pH lower than 7.0

Excipient stock solutions of L-histidine (200 mM), mannitol (500 mM) and m-cresol (1034 mg/ml) were prepared. Excipient solutions were mixed according to the compositions set out in Table 2, and stock solution of ZP1848 (approx. 40 mg/mL) were added. The volume was adjusted to 90% of final volume, pH was adjusted with acetic acid (200 mM) and/or L-arginine (200 to 250 mM), m-cresol was added, and the volume was adjusted to the final volume. The formulations were then sterile-filtered and filled in pharmaceutically acceptable containers, such as vials or cartridges.

The results of this experiment are shown in Table 2. This example confirmed that reducing the pH of the formulation, for example to a pH of less than 7.0, had the effect of improving the physical stability of formulations that include a preservative, reducing the precipitation of the active drug, ZP1848.

### Example 4: Stable formulations containing propylene glycol

Excipient stock solutions of L-histidine (200 mM), mannitol (500 mM), propylene glycol (500 mM) and m-cresol (1034 mg/mL) were prepared. Excipients solutions were mixed according to the desired composition (see Table), and stock solution of ZP1848 (approx. 40 mg/mL) was added. The volume was adjusted to 90% of final volume, the pH was adjusted with acetic acid (1 M) and/or L-arginine (200 or 250 mM), m-cresol was added, and the volume was adjusted to the final volume. The formulations were then sterile-filtered and filled in pharmaceutically acceptable containers, such as cartridges. The composition and physical stability at 5°C of ZP1848 formulations in cartridges was evaluated by examining the visual appearance of the formulation.

The results of this experiment are shown in Table 3.

While the present invention has been described in conjunction with the embodiments described above, many equivalent modifications and variations will be apparent to those skilled in the art when given this disclosure. Accordingly, the embodiments of the invention set forth are considered to be illustrative and not limiting.

**Table 1. Effect of m-cresol. Composition and physical stability at 5°C of ZP1848 formulations as evaluated by visual appearance.**

| **Formulation** | **pH** | **ZP1848 (mg/mL)** | **Mannitol (mM)** | **Histidine (mM)** | **Meta-cresol (mg/mL)** | **Other preservative agent** | **Visual appearance (clear/turbid/precipitated)** | | |
|---|---|---|---|---|---|---|---|---|---|
| | | | | | | | **0 weeks** | **5 weeks** | **13 weeks** |
| **1** | 7.0 | 10 | 230 | 15 | - | - | Clear | Clear | Clear |
| **2** | 7.0 | 10 | 230 | 15 | 3.15 | - | Clear | Precipitated | Precipitated |

**Table 2. Effect of lower pH. Composition and physical stability at 5°C of ZP1848 formulations as evaluated by visual appearance.**

| **Formulation** | **pH** | **ZP1848 (mg/mL)** | **Mannitol (mM)** | **Histidine (mM)** | **Meta-cresol (mg/mL)** | **Visual (clear/turbid/precipitated)** | | |
|---|---|---|---|---|---|---|---|---|
| | | | | | | **0 weeks** | **13 weeks** | **26 weeks** |
| **1** | 7 | 10 | 230 | 15 | 3.15 | Clear | Precipitated | Precipitated |
| **2** | 6.8 | 10 | 230 | 15 | 3.15 | Clear | Clear | Clear |
| **3** | 6.6 | 10 | 230 | 15 | 3.15 | Clear | Clear | Clear |
| **4** | 6.4 | 10 | 230 | 15 | 3.15 | Clear | Clear | Clear |
| **5** | 6.3 | 10 | 230 | 15 | 3.15 | Clear | Clear | Clear |
| **6** | 6.0 | 10 | 230 | 15 | 3.15 | Clear | Clear | Clear |
| **7** | 5.6 | 10 | 230 | 15 | 3.15 | Clear | Clear | Clear |

**Table 3. Effect of propylene glycol. Composition and physical stability at 5°C of ZP1848 formulations as evaluated by visual appearance.**

| **Formulation** | **pH** | **ZP1848 (mg/ml)** | **Mannitol [mM]** | **Histidine [mM]** | **Meta-cresol (mg/mL)** | **Propylene glycol (mM)** | **Visual appearance (clear/turbid)** | | |
|---|---|---|---|---|---|---|---|---|---|
| | | | | | | | **0 weeks** | **13 weeks** | **26 weeks** |
| **1** | 7 | 10 | 230 | 15 | 3.15 | - | Clear | Precipitated | Precipitated |
| **2** | 7 | 10 | 230 | 15 | - | - | Clear | Clear | Clear |
| **3** | 7 | 10 | 100 | 15 | 3.15 | 150 | Clear | Clear | Clear |
| **4** | 6.5 | 10 | 100 | 15 | 3.15 | 150 | Clear | Clear | Clear |
| **5** | 6 | 10 | 100 | 15 | 3.15 | 150 | Clear | Clear | Clear |
| **6** | 6 | 10 | 100 | 15 | 3.15 | 150 | Clear | Clear | Clear |
| **7** | 6 | 10 | 100 | 15 | 3.15 | 150 | Clear | Clear | Clear |
| **8** | 5.5 | 10 | 100 | 15 | 3.15 | 150 | Clear | Clear | Clear |
| **9** | 6 | 10 | 230 | 15 | 3.15 | - | Clear | Clear | Clear |
| **10** | 6 | 10 | 200 | 15 | 3.15 | 50 | Clear | Clear | Clear |
| **11** | 6 | 10 | 50 | 15 | 3.15 | 200 | Clear | Clear | Clear |

## Claims

1. A stable liquid pharmaceutical multi-dose formulation, the formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol or phenol as a preservative at a concentration of 1.0 to 5.0 mg/mL;
(c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(d) a non-ionic tonicity modifier selected from the group consisting of mannitol, sucrose, glycerol, sorbitol and trehalose at a concentration of about 20 mM to about 360 mM;
(e) a pH of about 5.0 to about 6.8.

2. A stable liquid pharmaceutical multi-dose formulation, the formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol or phenol as a preservative at a concentration of 1.0 to 5.0 mg/mL;
(c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(d) a non-ionic tonicity modifier selected from the group consisting of mannitol, sucrose, glycerol, sorbitol and trehalose at a concentration of about 20 mM to about 360 mM;
(e) a pH of about 5.5 to about 7.0; and
(f) propylene glycol at a concentration of 20 mM to 300 mM.

3. The formulation of claim 2, wherein the formulation comprises propylene glycol at a concentration of 20 mM to 300mM, optionally wherein the formulation comprises propylene glycol at a concentration of 50 mM to 200mM.

4. The formulation according to any one of claims 1 to 3, wherein the formulation is an aqueous formulation.

5. The formulation according to any one of claims 1 to 4, wherein the formulation is stable for at least 18 months when stored at 2-8°C.

6. The formulation according to any one of the preceding claims, wherein the GLP-2 analogue is present in the formulation at a concentration of about 1 mg/mL to about 20 mg/mL, optionally wherein the GLP-2 analogue is present in the formulation at a concentration of about 2 mg/mL to about 15 mg/mL.

7. The formulation according to any one of the preceding claims, wherein the formulation is a ready-to-use formulation.

8. The formulation according to any one of the preceding claims, wherein the GLP-2 analogue is present in the formulation at a concentration of about 2 mg/mL, 10 mg/mL or 20 mg/mL.

9. The formulation according to any one of the preceding claims, wherein the histidine buffer is present in the formulation at a concentration of about 5 mM to about 25 mM, optionally wherein the histidine buffer is present in the formulation at a concentration of about 15 mM.

10. The formulation according to any one of the preceding claims, wherein the mannitol is present in the formulation at a concentration of about 150 mM to about 250 mM, optionally wherein the mannitol is present in the formulation at a concentration of about 230 mM.

11. The formulation according to claim 1, wherein the formulation has a pH of about 6.2 to about 6.8.

12. The formulation according to claim 2, wherein the formulation has a pH of about 6.0 to about 7.0.

13. The formulation according to claim 1, wherein the formulation comprises:
(a) the GLP-2 analogue at a concentration of about 2 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM; and
(e) a pH of about 6.0 to about 6.8.

14. The formulation according to claim 1 or claim 13, wherein the formulation comprises:
(a) the GLP-2 analogue at a concentration of about 1mg/mL or about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 200 mM to about 250 mM; and
(e) a pH of about 6.0 to about 6.8.

15. The formulation according to any one of claims 1, 13 or 14, wherein the formulation consists of the GLP-2 analogue at a concentration of about 2 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

16. The formulation according to any one of claims 1, 13 or 14, wherein the formulation consists of the GLP-2 analogue at a concentration of about 2 mg/ml, about 10 mg/ml or about 20 mg/ml; a meta-cresol as a preservative at a concentration of 3.0 to 4.0 mg/ml; a histidine buffer at a concentration of about 10 mM to about 20 mM; mannitol as a non-ionic tonicity modifier at a concentration of about 200 mM to about 250 mM; the formulation having a pH of about 6.2 to about 6.8.

17. A stable liquid pharmaceutical multi-dose formulation, the formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 5 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM;
(e) a pH of about 5.0 to about 7.0; and
(f) propylene glycol at a concentration of 20 mM to 300 mM.

18. The formulation according to claim 17, wherein the formulation comprises the GLP-2 analogue at a concentration of about 20 mg/mL.

19. A stable liquid pharmaceutical multi-dose formulation, the formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 50 mM to about 230 mM; and
(e) a pH of about 5.0 to about 7.0; and
(f) propylene glycol at a concentration of 50 mM to 200 mM.

20. The formulation according to claim 18 or claim 19, wherein the formulation consists of the GLP-2 analogue at a concentration of about 10 mg/mL; meta-cresol as a preservative at a concentration of 3.0 to 4.0 mg/mL; histidine buffer at a concentration of about 10 mM to about 20 mM; mannitol as a non-ionic tonicity modifier at a concentration of about 50 mM to about 230 mM; and propylene glycol at a concentration of 50 mM to 200 mM, the formulation having a pH of about 5.0 to about 7.0.

21. The formulation according to any one of the preceding claims, wherein the histidine buffer is L-histidine and/or the mannitol is D-mannitol.

22. The formulation according to any one of the preceding claims, wherein the formulation is stable at 2-8°C for at least 6 months, at least 12 months, at least 18 months or at least 24 months, optionally wherein the GLP-2 analogue in the formulation retains at least about 90% of its biological activity after 18 months of storage 2-8°C.

23. The formulation according to any one of the preceding claims which is sterile.

24. The formulation according to any one of the preceding claims, wherein the formulation is administration to a subject by injection, optionally wherein the injection is subcutaneous injection.

25. The formulation according to any one of the preceding claims, wherein the GLP-2 analogue is provided as an acetate salt, optionally wherein the GLP-2 analogue is ZP1848 or ZP1848-acetate, optionally wherein the acetate salt of the GLP-2 analogue is represented by the formula (GLP-2 analogue), x(CH₃COOH) where x is 1.0 to 8.0.

26. An article of manufacture or a kit comprising a container holding the stable pharmaceutical formulation of any one of claims 1 to 24.

27. A delivery device containing a liquid formulation comprising a GLP-2 analogue of any one of claims 1 to 25, optionally wherein the delivery device is pre-filled syringe, an injector device, an injector pen, an adjustable dose auto-injector, a disposable auto-injector, a wearable injector, or an infusion pump.

28. A formulation of the glucagon-like peptide 2 (GLP-2) analogue of any one of claims 1 to 25 for use in therapy.

29. A formulation of the glucagon-like peptide 2 (GLP-2) analogue of any one of claims 1 to 25 for use in a method for the treatment and/or prevention of a stomach and/or bowel-related disorder in a human patient.

30. The formulation of the glucagon-like peptide 2 (GLP-2) analogue for use in the method of treatment and/or prevention of claim 29, wherein the stomach and/or bowel-related disorder is ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS), optionally wherein the stomach and/or bowel-related disorder is short bowel syndrome.

31. The formulation of the glucagon-like peptide 2 (GLP-2) analogue for use in a method for the treatment according to claim 29, wherein the stomach and/or bowel-related disorder is radiation enteritis, infectious or post-infectious enteritis, or small intestinal damage due to toxic or other chemotherapeutic agents, optionally wherein treatment with the GLP-2 analogue is combined with one or more anti-cancer therapies and/or treatment the anti-cancer therapy comprises administering one or more chemotherapeutic agent(s) to the patient or treating the patient with radiation therapy.

32. The formulation of the glucagon-like peptide 2 (GLP-2) analogue for use in a method for the treatment according to claim 31, wherein the formulation is used in the treatment and/or prevention of a side effect of chemotherapy or radiation treatment, optionally wherein the side effect of chemotherapy is diarrhoea, abdominal cramping, vomiting or structural and functional damage of the intestinal epithelium resulting from chemotherapy treatment.

33. The formulation of the glucagon-like peptide 2 (GLP-2) analogue for use in a method for the treatment according to any one of claims 30 to 32, wherein the human patient is a patient having SBS-intestinal failure.

34. The formulation of the glucagon-like peptide 2 (GLP-2) analogue for use in a method for the treatment according to any one of claims 30 to 33, wherein the human patient is a patient being on the border between being a patient having SBS-intestinal insufficiency and SBS-intestinal failure.

35. The formulation of the glucagon-like peptide 2 (GLP-2) analogue for use in a method for the treatment according to any one of claims 28 to 34, wherein the method comprises administering the GLP-2 analogue to the patient once or twice daily, or wherein the method comprises administering the GLP-2 analogue to the patient once or twice weekly.

36. Use of a formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing an liquid pharmaceutical formulation which is stable for 18 months when stored at 2-8°C, wherein the formulation comprises:
(a) the GLP-2 analogue at a concentration of about 1 mg/mL to about 30 mg/mL;
(b) meta-cresol or phenol as a preservative at a concentration of 1.0 to 5.0 mg/mL;
(c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(d) a non-ionic tonicity modifier selected from the group consisting of mannitol, sucrose, glycerol, sorbitol and trehalose at a concentration of about 20 mM to about 360 mM;
(e) a pH of about 5.0 to about 6.8.

37. Use of a formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
for providing an liquid pharmaceutical formulation which is stable for 18 months when stored at 2-8°C, wherein the formulation comprises:
wherein the formulation comprises:
(a) the GLP-2 analogue at a concentration of about 1 mg/mL to about 30 mg/mL;
(b) meta-cresol or phenol as a preservative at a concentration of 1.0 to 5.0 mg/mL;
(c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(d) a non-ionic tonicity modifier selected from the group consisting of mannitol, sucrose, glycerol, sorbitol and trehalose at a concentration of about 20 mM to about 360 mM;
(e) a pH of about 5.5 to about 7.0; and
(f) propylene glycol at a concentration of 20 mM to 300 mM .

38. A process for producing a stable liquid pharmaceutical formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu- Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys;
or a pharmaceutically acceptable salt or derivative thereof;
wherein the process comprising formulating (a) the GLP-2 analogue at a concentration of about 1 mg/mL to about 30 mg/mL; (b) meta-cresol or phenol as a preservative at a concentration of 1.0 to 5.0 mg/mL; (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (d) a non-ionic tonicity modifier selected from the group consisting of mannitol, sucrose, glycerol, sorbitol and trehalose at a concentration of about 20 mM to about 360 mM; at a pH of about 5.0 to about 6.8.

39. A process for producing a stable liquid pharmaceutical formulation comprising a glucagon-like peptide 2 (GLP-2) analogue, wherein the GLP-2 analogue is represented by the formula:
R¹-Z¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 1 mg/mL to about 30 mg/mL; (b) meta-cresol or phenol as a preservative at a concentration of 1.0 to 5.0 mg/mL; (c) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (d) a non-ionic tonicity modifier selected from the group consisting of mannitol, sucrose, glycerol, sorbitol and trehalose at a concentration of about 20 mM to about 360 mM; (e) propylene glycol at a concentration of 20 mM to 300 mM ; at a pH of about 5.5 to about 7.0.

## Patentansprüche

1. Stabile, flüssige pharmazeutische Mehrdosenformulierung, wobei die Formulierung ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei die Formulierung Folgendes umfasst:
(a) das GLP-2-Analogon in einer Konzentration von etwa 1 mg/ml bis etwa 30 mg/ml;
(b) ein meta-Cresol oder Phenol als Konservierungsmittel in einer Konzentration von 1,0 bis 5,0 mg/ml;
(c) einen Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist;
(d) einen nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM;
(e) einen pH von etwa 5,0 bis etwa 6,8.

2. Stabile, flüssige pharmazeutische Mehrdosenformulierung, wobei die Formulierung ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei die Formulierung Folgendes umfasst:
(a) das GLP-2-Analogon in einer Konzentration von etwa 1 mg/ml bis etwa 30 mg/ml;
(b) ein meta-Cresol oder Phenol als Konservierungsmittel in einer Konzentration von 1,0 bis 5,0 mg/ml;
(c) einen Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist;
(d) einen nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM;
(e) einen pH von etwa 5,5 bis etwa 7,0; und
(f) Propylenglykol in einer Konzentration von 20 mM bis 300 mM.

3. Formulierung nach Anspruch 2, wobei die Formulierung Propylenglykol in einer Konzentration von 20 mM bis 300 mM umfasst, wobei die Formulierung gegebenenfalls Propylenglykol in einer Konzentration von 50 mM bis 200 mM umfasst.

4. Formulierung nach einem der Ansprüche 1 bis 3, wobei die Formulierung eine wässrige Formulierung ist.

5. Formulierung nach einem der Ansprüche 1 bis 4, wobei die Formulierung zumindest 18 Monate lang stabil ist, wenn sie bei 2-8 °C gelagert wird.

6. Formulierung nach einem der vorangegangenen Ansprüche, wobei das GLP-2-Analogon in der Formulierung in einer Konzentration von etwa 1 mg/ml bis etwa 20 mg/ml vorhanden ist, wobei das GLP-2-Analogon gegebenenfalls in der Formulierung in einer Konzentration von etwa 2 mg/ml bis etwa 15 mg/ml vorhanden ist.

7. Formulierung nach einem der vorangegangenen Ansprüche, wobei die Formulierung eine gebrauchsfertige Formulierung ist.

8. Formulierung nach einem der vorangegangenen Ansprüche, wobei das GLP-2-Analogon in der Formulierung in einer Konzentration von etwa 2 mg/ml, 10 mg/ml oder 20 mg/ml vorhanden ist.

9. Formulierung nach einem der vorangegangenen Ansprüche, wobei der Histidinpuffer in der Formulierung in einer Konzentration von etwa 5 mM bis etwa 25 mM vorhanden ist, wobei der Histidinpuffer gegebenenfalls in der Formulierung in einer Konzentration von etwa 15 mM vorhanden ist.

10. Formulierung nach einem der vorangegangenen Ansprüche, wobei der Mannit in der Formulierung in einer Konzentration von etwa 150 mM bis etwa 250 mM vorhanden ist, wobei der Mannit gegebenenfalls in der Formulierung in einer Konzentration von etwa 230 mM vorhanden ist.

11. Formulierung nach Anspruch 1, wobei die Formulierung einen pH von etwa 6,2 bis etwa 6,8 aufweist.

12. Formulierung nach Anspruch 2, wobei die Formulierung einen pH von etwa 6,0 bis etwa 7,0 aufweist.

13. Formulierung nach Anspruch 1, wobei die Formulierung Folgendes umfasst:
(a) das GLP-2-Analogon in einer Konzentration von etwa 2 mg/ml bis etwa 20 mg/ml;
(b) ein meta-Cresol als Konservierungsmittel in einer Konzentration von 1,0 bis 5,0 mg/ml;
(c) einen Histidinpuffer in einer Konzentration von etwa 5 mM bis etwa 50 mM;
(d) Mannit als nichtionischen Tonizitätsmodifikator in einer Konzentration von etwa 20 mM bis etwa 360 mM; und
(e) einen pH von etwa 6,0 bis etwa 6,8.

14. Formulierung nach Anspruch 1 oder Anspruch 13, wobei die Formulierung Folgendes umfasst:
(a) das GLP-2-Analogon in einer Konzentration von etwa 1 mg/ml oder etwa 10 mg/ml;
(b) ein meta-Cresol als Konservierungsmittel in einer Konzentration von 3,0 bis 4,0 mg/ml;
(c) einen Histidinpuffer in einer Konzentration von etwa 10 mM bis etwa 20 mM;
(d) Mannit als nichtionischen Tonizitätsmodifikator in einer Konzentration von etwa 200 mM bis etwa 250 mM; und
(e) einen pH von etwa 6,0 bis etwa 6,8.

15. Formulierung nach einem der Ansprüche 1, 13 oder 14, wobei die Formulierung aus dem GLP-2-Analogon in einer Konzentration von etwa 2 mg/ml oder etwa 10 mg/ml; einem meta-Cresol als Konservierungsmittel in einer Konzentration von 3,0 bis 4,0 mg/ml; einem Histidinpuffer in einer Konzentration von etwa 10 mM bis etwa 20 mM; Mannit als nichtionischem Tonizitätsmodifikator in einer Konzentration von etwa 200 mM bis etwa 250 mM besteht; wobei die Formulierung einen pH von etwa 6,0 bis etwa 6,8 aufweist.

16. Formulierung nach einem der Ansprüche 1, 13 oder 14, wobei die Formulierung aus dem GLP-2-Analogon in einer Konzentration von etwa 2 mg/ml, etwa 10 mg/ml oder etwa 20 mg/ml; einem meta-Cresol als Konservierungsmittel in einer Konzentration von 3,0 bis 4,0 mg/ml; einem Histidinpuffer in einer Konzentration von etwa 10 mM bis etwa 20 mM; Mannit als nichtionischem Tonizitätsmodifikator in einer Konzentration von etwa 200 mM bis etwa 250 mM besteht; wobei die Formulierung einen pH von etwa 6,2 bis etwa 6,8 aufweist.

17. Stabile, flüssige pharmazeutische Mehrdosenformulierung, wobei die Formulierung ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei die Formulierung Folgendes umfasst:
(a) das GLP-2-Analogon in einer Konzentration von etwa 5 mg/ml bis etwa 20 mg/ml;
(b) ein meta-Cresol als Konservierungsmittel in einer Konzentration von 1,0 bis 5,0 mg/ml;
(c) einen Histidinpuffer in einer Konzentration von etwa 5 mM bis etwa 50 mM;
(d) Mannit als nichtionischen Tonizitätsmodifikator in einer Konzentration von etwa 20 mM bis etwa 360 mM;
(e) einen pH von etwa 5,0 bis etwa 7,0; und
(f) Propylenglykol in einer Konzentration von 20 mM bis 300 mM.

18. Formulierung nach Anspruch 17, wobei die Formulierung das GLP-2-Analogon in einer Konzentration von etwa 20 mg/ml umfasst.

19. Stabile, flüssige pharmazeutische Mehrdosenformulierung, wobei die Formulierung ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei die Formulierung Folgendes umfasst:
(a) das GLP-2-Analogon in einer Konzentration von etwa 10 mg/ml;
(b) ein meta-Cresol als Konservierungsmittel in einer Konzentration von 3,0 bis 4,0 mg/ml;
(c) einen Histidinpuffer in einer Konzentration von etwa 10 mM bis etwa 20 mM;
(d) Mannit als nichtionischen Tonizitätsmodifikator in einer Konzentration von etwa 50 mM bis etwa 230 mM;
(e) einen pH von etwa 5,0 bis etwa 7,0; und
(f) Propylenglykol in einer Konzentration von 50 bis 200 mM.

20. Formulierung nach Anspruch 18 oder Anspruch 19, wobei die Formulierung aus dem GLP-2-Analogon in einer Konzentration von etwa 10 mg/ml; einem meta-Cresol als Konservierungsmittel in einer Konzentration von 3,0 bis 4,0 mg/ml; einem Histidinpuffer in einer Konzentration von etwa 10 mM bis etwa 20 mM; Mannit als nichtionischem Tonizitätsmodifikator in einer Konzentration von etwa 50 mM bis etwa 230 mM; und Propylenglykol in einer Konzentration von 50 mM bis 200 mM besteht; wobei die Formulierung einen pH von etwa 5,0 bis etwa 7,0 aufweist.

21. Formulierung nach einem der vorangegangenen Ansprüche, wobei der Histidinpuffer L-Histidin ist und/oder der Mannit D-Mannit ist.

22. Formulierung nach einem der vorangegangenen Ansprüche, wobei die Formulierung bei 2-8 °C zumindest 6 Monate, zumindest 12 Monate, zumindest 18 Monate oder zumindest 24 Monate lang stabil ist, wobei das GLP-2-Analogon in der Formulierung nach 18 Monaten Lagerung bei 2-8 °C gegebenenfalls zumindest etwa 90 % seiner biologischen Aktivität beibehält.

23. Formulierung nach einem der vorangegangenen Ansprüche, die steril ist.

24. Formulierung nach einem der vorangegangenen Ansprüche, wobei die Formulierung einem Individuum mittels Injektion verabreicht wird, wobei die Injektion gegebenenfalls eine subkutane Injektion ist.

25. Formulierung nach einem der vorangegangenen Ansprüche, wobei das GLP-2-Analogon als Acetatsalz bereitgestellt ist, wobei das GLP-2-Analogon gegebenenfalls ZP1848 oder ZP1848-Acetat ist, wobei das Acetatsalz des GLP-2-Analogons gegebenenfalls durch die Formel (GLP-2-Analogon), x(CH₃COOH) dargestellt ist, worin x = 1,0 bis 8,0 ist.

26. Fabrikat oder Set, das einen Behälter umfasst, der eine stabile pharmazeutische Formulierung nach einem der Ansprüche 1 bis 24 enthält.

27. Abgabevorrichtung, die eine flüssige Formulierung, die ein GLP-2-Analogon umfasst, nach einem der Ansprüche 1 bis 25 enthält, wobei die Abgabevorrichtung gegebenenfalls eine vorgefüllte Spritze, eine Injektionsvorrichtung, ein Injektionspen, ein Autoinjektor mit einstellbarer Dosis, ein Einweg-Autoinjektor, ein tragbarer Injektor oder eine Infusionspumpe ist.

28. Formulierung eines Analogons eines Glucagon-ähnlichen Peptids 2 (GLP-2) nach einem der Ansprüche 1 bis 25 zur Verwendung in einer Therapie.

29. Formulierung eines Analogons eines Glucagon-ähnlichen Peptids 2 (GLP-2) nach einem der Ansprüche 1 bis 25 zur Verwendung in einem Behandlungs- und/oder Präventionsverfahren eines magen- und/oder darmbezogenen Leidens bei einem menschlichen Patienten.

30. Formulierung eines Analogons eines Glucagon-ähnlichen Peptids 2 (GLP-2) zur Verwendung in einem Behandlungs- und/oder Präventionsverfahren nach Anspruch 29, wobei das magen- und/oder darmbezogene Leiden ein Geschwür, eine Verdauungsstörung, ein Malabsorptionssyndrom, ein Kurzdarmsyndrom, ein Cul-de-sac-Syndrom, eine entzündliche Darmerkrankung, Zöliakie (z. B. infolge einer gluteninduzierten Enteropathie oder Zöliakie), tropische Sprue, hypogammaglobulinämische Sprue, Enteritis, regionale Enteritis (Morbus Crohn), Colitis ulcerosa, ein Dünndarmschaden oder ein Kurzdarmsyndrom (SBS) ist, wobei das magen- und/oder darmbezogene gegebenenfalls ein Kurzdarmsyndrom ist.

31. Formulierung eines Analogons eines Glucagon-ähnlichen Peptids 2 (GLP-2) zur Verwendung in einem Behandlungsverfahren nach Anspruch 29, wobei das magen- und/oder darmbezogene Leiden Strahlenenteritis, eine infektiöse oder postinfektiöse Enteritis oder eine Schädigung des Dünndarms durch toxische oder andere chemotherapeutische Wirkstoffe ist, wobei eine Behandlung mit dem GLP-2-Analogon gegebenenfalls mit einer oder mehreren Antikrebstherapien kombiniert wird und/oder die Antikrebstherapie die Verabreichung eines oder mehrerer chemotherapeutischer Wirkstoffe an den Patienten oder das Behandeln des Patienten mit Strahlentherapie umfasst.

32. Formulierung eines Analogons eines Glucagon-ähnlichen Peptids 2 (GLP-2) zur Verwendung in einem Behandlungsverfahren nach Anspruch 31, wobei die Formulierung bei der Behandlung und/oder Prävention einer Nebenwirkung einer Chemotherapie oder Strahlenbehandlung verwendet wird, wobei die Nebenwirkung der Chemotherapie gegebenenfalls Diarrhoe, Magenkrämpfe, Erbrechen oder eine strukturelle und funktionelle Schädigung des Darmepithels aufgrund der chemotherapeutischen Behandlung ist.

33. Formulierung eines Analogons eines Glucagon-ähnlichen Peptids 2 (GLP-2) zur Verwendung in einem Behandlungsverfahren nach einem der Ansprüche 30 bis 32, wobei der menschliche Patient ein Patient mit SBS-Darmversagen ist.

34. Formulierung eines Analogons eines Glucagon-ähnlichen Peptids 2 (GLP-2) zur Verwendung in einem Behandlungsverfahren nach einem der Ansprüche 30 bis 33, wobei der menschliche Patient ein Patient ist, der sich an der Grenze zwischen einem Patienten mit SBS-Darminsuffizienz und SBS-Darmversagen befindet.

35. Formulierung eines Analogons eines Glucagon-ähnlichen Peptids 2 (GLP-2) zur Verwendung in einem Behandlungsverfahren nach einem der Ansprüche 28 bis 34, wobei das Verfahren die Verabreichung des GLP-2-Analogons an den Patienten einmal oder zweimal täglich umfasst oder wobei das Verfahren die Verabreichung des GLP-2-Analogons an den Patienten einmal oder zweimal wöchentlich umfasst.

36. Verwendung einer Formulierung, die ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
zur Bereitstellung einer flüssigen pharmazeutischen Formulierung, die 18 Monate lang stabil ist, wenn sie bei 2-8 °C gelagert wird, wobei die Formulierung Folgendes umfasst:
(a) das GLP-2-Analogon in einer Konzentration von etwa 1 mg/ml bis etwa 30 mg/ml;
(b) ein meta-Cresol oder Phenol als Konservierungsmittel in einer Konzentration von 1,0 bis 5,0 mg/ml;
(c) einen Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist;
(d) einen nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM;
(e) einen pH von etwa 5,0 bis etwa 6,8.

37. Verwendung einer Formulierung, die ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
zum Bereitstellen einer flüssigen pharmazeutischen Formulierung, die 18 Monate lang stabil ist, wenn sie bei 2-8 °C gelagert wird, wobei die Formulierung Folgendes umfasst:
(a) das GLP-2-Analogon in einer Konzentration von etwa 1 mg/ml bis etwa 30 mg/ml;
(b) ein meta-Cresol oder Phenol als Konservierungsmittel in einer Konzentration von 1,0 bis 5,0 mg/ml;
(c) einen Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist;
(d) einen nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM;
(e) einen pH von etwa 5,5 bis etwa 7,0; und
(f) Propylenglykol in einer Konzentration von 20 mM bis 300 mM.

38. Verfahren zur Herstellung einer stabilen, flüssigen pharmazeutischen Formulierung, die ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei das Verfahren das Formulieren von (a) dem GLP-2-Analogon in einer Konzentration von etwa 1 mg/ml bis etwa 30 mg/ml; (b) einem meta-Cresol oder Phenol als Konservierungsmittel in einer Konzentration von 1,0 bis 5,0 mg/ml; (c) einem Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist; (d) einem nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM; bei einem pH von etwa 5,0 bis etwa 6,8 umfasst.

39. Verfahren zur Herstellung einer stabilen, flüssigen pharmazeutischen Formulierung, die ein Analogon eines Glucagon-ähnlichen Peptids 2 (GLP-2), wobei das GLP-2-Analogon durch die folgende Formel dargestellt ist:
R¹-Z¹-His-Gly-Glu-Gly-X5-Phe-Ser-Ser-Glu-Leu-X11-Thr-Ile-Leu-Asp-Ala-Leu-Ala- Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z¹ und Z² unabhängig voneinander fehlen oder eine Peptidsequenz aus 1-6 Aminosäureeinheiten Lys sind;
oder ein pharmazeutisch annehmbares Salz oder Derivat davon umfasst;
wobei das Verfahren das Formulieren von (a) dem GLP-2-Analogon in einer Konzentration von etwa 1 mg/ml bis etwa 30 mg/ml; (b) einem meta-Cresol oder Phenol als Konservierungsmittel in einer Konzentration von 1,0 bis 5,0 mg/ml; (c) einem Puffer, der aus der aus einem Histidinpuffer, Mesylatpuffer, Acetatpuffer, Glycinpuffer, Lysinpuffer, TRIS-Puffer, Bis-Tris-Puffer und MOPS-Puffer bestehenden Gruppe ausgewählt ist, wobei der Puffer in einer Konzentration von etwa 5 mM bis etwa 50 mM vorhanden ist; (d) einem nichtionischen Tonizitätsmodifikator, der aus der aus Mannit, Saccharose, Glycerin, Sorbit und Trehalose bestehenden Gruppe ausgewählt ist, in einer Konzentration von etwa 20 mM bis etwa 360 mM; (e) Propylenglykol in einer Konzentration von 20 mM bis 300 mM; bei einem pH von etwa 5,5 bis etwa 7,0 umfasst.

## Revendications

1. Formulation pharmaceutique multidose liquide stable, la formulation comprenant un analogue du peptide-2 de type glucagon (GLP-2), dans laquelle l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
où :
R¹ est hydrogène, alkyle en C₁₋₄ (par exemple, méthyle), acétyle, formyle, benzoyle ou trifluoroacétyle ;
X5 est Ser ou Thr ;
X11 est Ala ou Ser ;
R² est NH₂ ou OH ; et
Z¹ et Z² sont indépendamment absents ou une séquence peptidique de 1 à 6 motifs d'acide aminé Lys ;
ou un sel ou dérivé pharmaceutiquement acceptable de celuici ;
la formulation comprenant :
(a) l'analogue de GLP-2 à une concentration d'environ 1 mg/ml à environ 30 mg/ml ;
(b) du méta-crésol ou du phénol en tant que conservateur à une concentration de 1,0 à 5,0 mg/ml ;
(c) un tampon choisi dans le groupe constitué d'un tampon histidine, un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ;
(d) un modificateur de tonicité non ionique choisi dans le groupe constitué du mannitol, du saccharose, du glycérol, du sorbitol et du tréhalose à une concentration d'environ 20 mM à environ 360 mM ;
(e) un pH d'environ 5,0 à environ 6,8.

2. Formulation pharmaceutique multidose liquide stable, la formulation comprenant un analogue du peptide-2 de type glucagon (GLP-2), dans laquelle l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
où :
R¹ est hydrogène, alkyle en C₁₋₄ (par exemple, méthyle), acétyle, formyle, benzoyle ou trifluoroacétyle ;
X5 est Ser ou Thr ;
X11 est Ala ou Ser ;
R² est NH₂ ou OH ; et
Z¹ et Z² sont indépendamment absents ou une séquence peptidique de 1 à 6 motifs d'acide aminé Lys ;
ou un sel ou dérivé pharmaceutiquement acceptable de celuici ;
la formulation comprenant :
(a) l'analogue de GLP-2 à une concentration d'environ 1 mg/ml à environ 30 mg/ml ;
(b) du méta-crésol ou du phénol en tant que conservateur à une concentration de 1,0 à 5,0 mg/ml ;
(c) un tampon choisi dans le groupe constitué d'un tampon histidine, un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ;
(d) un modificateur de tonicité non ionique choisi dans le groupe constitué du mannitol, du saccharose, du glycérol, du sorbitol et du tréhalose à une concentration d'environ 20 mM à environ 360 mM ;
(e) un pH d'environ 5,5 à environ 7,0 ; et
(f) du propylène glycol à une concentration de 20 mM à 300 mM.

3. Formulation selon la revendication 2, la formulation comprenant du propylène glycol à une concentration de 20 mM à 300 mM, la formulation comprenant facultativement du propylène glycol à une concentration de 50 mM à 200 mM.

4. Formulation selon l'une quelconque des revendications 1 à 3, la formulation étant une formulation aqueuse.

5. Formulation selon l'une quelconque des revendications 1 à 4, la formulation étant stable pendant au moins 18 mois conservée à 2 à 8 °C.

6. Formulation selon l'une quelconque des revendications précédentes, dans laquelle l'analogue de GLP-2 est présent dans la formulation à une concentration d'environ 1 mg/ml à environ 20 mg/ml, facultativement dans laquelle l'analogue de GLP-2 est présent dans la formulation à une concentration d'environ 2 mg/ml à environ 15 mg/ml.

7. Formulation selon l'une quelconque des revendications précédentes, la formulation étant une formulation prête à l'usage.

8. Formulation selon l'une quelconque des revendications précédentes, dans laquelle l'analogue de GLP-2 est présent dans la formulation à une concentration d'environ 2 mg/ml, 10 mg/ml ou 20 mg/ml.

9. Formulation selon l'une quelconque des revendications précédentes, dans laquelle l'analogue de GLP-2 est présent dans la formulation à une concentration d'environ 5 mM à environ 25 mM, facultativement dans laquelle l'analogue de GLP-2 est présent dans la formulation à une concentration d' environ 15 mM.

10. Formulation selon l'une quelconque des revendications précédentes, dans laquelle le mannitol est présent dans la formulation à une concentration d'environ 150 mM à environ 250 mM, facultativement dans laquelle le mannitol est présent dans la formulation à une concentration d'environ 230 mM.

11. Formulation selon la revendication 1, la formulation ayant un pH d'environ 6,2 à environ 6,8.

12. Formulation selon la revendication 2, la formulation ayant un pH d'environ 6,0 à environ 7,0.

13. Formulation selon la revendication 1, la formulation comprenant :
(a) l'analogue de GLP-2 à une concentration d'environ 2 mg/ml à environ 20 mg/ml ;
(b) du méta-crésol en tant que conservateur à une concentration de 1,0 à 5,0 mg/ml ;
(c) un tampon histidine à une concentration d'environ 5 mM à environ 50 mM ;
(d) du mannitol en tant que modificateur de tonicité non ionique à une concentration d'environ 20 mM à environ 360 mM ; et
(e) un pH d'environ 6,0 à environ 6,8.

14. Formulation selon la revendication 1 ou la revendication 13, la formulation comprenant :
(a) l'analogue de GLP-2 à une concentration d'environ 1 mg/ml à environ 30 mg/ml ;
(b) du méta-crésol en tant que conservateur à une concentration de 3,0 à 4,0 mg/ml ;
(c) un tampon histidine à une concentration d'environ 10 mM à environ 20 mM ;
(d) du mannitol en tant que modificateur de tonicité non ionique à une concentration d'environ 200 mM à environ 250 mM ; et
(e) un pH d'environ 6,0 à environ 6,8.

15. Formulation selon l'une quelconque des revendications 1, 13 ou 14, la formulation étant constituée de l'analogue de GLP-2 à une concentration d'environ 2 mg/ml ou d'environ 10 mg/ml ; un méta-crésol en tant que conservateur à une concentration de 3,0 à 4,0 mg/ml ; un tampon histidine à une concentration d'environ 10 mM à environ 20 mM ; le mannitol en tant que modificateur de tonicité non ionique à une concentration d'environ 200 mM à environ 250 mM ; la formulation ayant un pH d'environ 6,0 à environ 6,8.

16. Formulation selon l'une quelconque des revendications 1, 13 ou 14, la formulation étant constituée de l'analogue de GLP-2 à une concentration d'environ 2 mg/ml ou d'environ 10 mg/ml ; un méta-crésol en tant que conservateur à une concentration de 3,0 à 4,0 mg/ml ; un tampon histidine à une concentration d'environ 10 mM à environ 20 mM ; le mannitol en tant que modificateur de tonicité non ionique à une concentration d'environ 200 mM à environ 250 mM ; la formulation ayant un pH d'environ 6,2 à environ 6,8.

17. Formulation pharmaceutique multidose liquide stable, la formulation comprenant un analogue du peptide-2 de type glucagon (GLP-2), dans laquelle l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
où :
R¹ est hydrogène, alkyle en C₁₋₄ (par exemple, méthyle), acétyle, formyle, benzoyle ou trifluoroacétyle ;
X5 est Ser ou Thr ;
X11 est Ala ou Ser ;
R² est NH₂ ou OH ; et
Z¹ et Z² sont indépendamment absents ou une séquence peptidique de 1 à 6 motifs d'acide aminé Lys ;
ou un sel ou dérivé pharmaceutiquement acceptable de celuici ;
la formulation comprenant :
(a) l'analogue de GLP-2 à une concentration d'environ 5 mg/ml à environ 20 mg/ml ;
(b) du méta-crésol en tant que conservateur à une concentration de 1,0 à 5,0 mg/ml ;
(c) un tampon histidine à une concentration d'environ 5 mM à environ 50 mM ;
(d) du mannitol en tant que modificateur de tonicité non ionique à une concentration d'environ 20 mM à environ 360 mM ; et
(e) un pH d'environ 5,0 à environ 7,0 ; et
(f) du propylène glycol à une concentration de 20 mM à 300 mM.

18. Formulation selon la revendication 17, la formulation comprenant l'analogue de GLP-2 à une concentration d'environ 20 mg/ml.

19. Formulation pharmaceutique multidose liquide stable, la formulation comprenant un analogue du peptide-2 de type glucagon (GLP-2), dans laquelle l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
où :
R¹ est hydrogène, alkyle en C₁₋₄ (par exemple, méthyle), acétyle, formyle, benzoyle ou trifluoroacétyle ;
X5 est Ser ou Thr ;
X11 est Ala ou Ser ;
R² est NH₂ ou OH ; et
Z¹ et Z² sont indépendamment absents ou une séquence peptidique de 1 à 6 motifs d'acide aminé Lys ;
ou un sel ou dérivé pharmaceutiquement acceptable de celuici ;
la formulation comprenant :
(a) l'analogue de GLP-2 à une concentration d'environ 10 mg/ml ;
(b) du méta-crésol en tant que conservateur à une concentration de 3,0 à 4,0 mg/ml ;
(c) un tampon histidine à une concentration d'environ 10 mM à environ 20 mM ;
(d) du mannitol en tant que modificateur de tonicité non ionique à une concentration d'environ 50 mM à environ 230 mM ; et
(e) un pH d'environ 5,0 à environ 7,0 ; et
(f) du propylène glycol à une concentration de 50 mM à 200 mM.

20. Formulation selon la revendication 18 ou la revendication 19, la formulation étant constituée de l'analogue de GLP-2 à une concentration d'environ 10 mg/ml ; du méta-crésol en tant que conservateur à une concentration de 3,0 à 4,0 mg/ml ; un tampon histidine à une concentration d'environ 10 mM à environ 20 mM ; du mannitol en tant que modificateur de tonicité non ionique à une concentration d'environ 50 mM à environ 230 mM ; et du propylène glycol à une concentration de 50 mM à 200 mM, la formulation ayant un pH d'environ 5,0 à environ 7,0.

21. Formulation selon l'une quelconque des revendications précédentes, dans laquelle le tampon histidine est la L-histidine et/ou le mannitol est le D-mannitol.

22. Formulation selon l'une quelconque des revendications précédentes, la formulation étant stable à 2 à 8 °C pendant au moins 6 mois, au moins 12 mois, au moins 18 mois ou au moins 24 mois, facultativement dans laquelle l'analogue de GLP-2 dans la formulation conserve au moins environ 90 % de son activité biologique après 18 mois de conservation à 2 à 8 °C.

23. Formulation selon l'une quelconque des revendications précédentes qui est stérile.

24. Formulation selon l'une quelconque des revendications précédentes, la formulation étant administrée à un sujet par injection, l'injection étant facultativement une injection sous-cutanée.

25. Formulation selon l'une quelconque des revendications précédentes, dans laquelle l'analogue de GLP-2 est fourni sous la forme d'un sel d'acétate, facultativement dans laquelle l'analogue de GLP-2 est ZP1848 ou l'acétate de ZP1848, facultativement dans laquelle le sel d'acétate de l'analogue de GLP-2 est représenté par la formule (analogue de GLP-2), x(CH₃COOH) où x est de 1,0 à 8,0.

26. Article manufacturé ou kit comprenant un récipient contenant la formulation pharmaceutique stable selon l'une quelconque des revendications 1 à 24.

27. Dispositif d'administration contenant une formulation liquide comprenant un analogue de GLP-2 selon l'une quelconque des revendications 1 à 25, le dispositif d'administration étant facultativement une seringue préremplie, un dispositif injecteur, un stylo injecteur, un auto-injecteur à dose réglable, un auto-injecteur jetable, un injecteur portable ou une pompe à perfusion.

28. Formulation de l'analogue du peptide-2 de type glucagon (GLP-2) selon l'une quelconque des revendications 1 à 25, destinée à être utilisée en thérapie.

29. Formulation de l'analogue du peptide-2 de type glucagon (GLP-2) selon l'une quelconque des revendications 1 à 25, destinée à être utilisée dans un procédé de traitement et/ou de prévention d'un trouble lié à l'estomac et/ou à l'intestin chez un patient humain.

30. Formulation de l'analogue du peptide-2 de type glucagon (GLP-2) destinée à être utilisée dans le procédé de traitement et/ou de prévention selon la revendication 29, le trouble lié à l'estomac et/ou l'intestin étant des ulcères, des troubles de la digestion, des syndromes de malabsorption, le syndrome de l'intestin court, le syndrome du cul-de-sac, une maladie inflammatoire de l'intestin, la sprue coeliaque (par exemple résultant d'une entéropathie induite par le gluten ou une maladie coeliaque), la sprue tropicale, la sprue hypogammaglobulinémique, une entérite, une entérite régionale (maladie de Crohn), la rectocolite hémorragique, une lésion de l'intestin grêle ou le syndrome de l'intestin court (SBS), le trouble lié à l'estomac et/ou l'intestin étant facultativement le syndrome de l'intestin court.

31. Formulation de l'analogue du peptide-2 de type glucagon (GLP-2) destinée à être utilisée dans un procédé de traitement selon la revendication 29, le trouble lié à l'estomac et/ou l'intestin étant une entérite due aux rayonnements, une entérite infectieuse ou post-infectieuse, ou une lésion de l'intestin grêle due à des agents chimiothérapeutiques toxiques ou autres, le traitement par l'analogue de GLP-2 étant facultativement combiné avec une ou plusieurs thérapies anticancéreuses et/ou la thérapie anticancéreuse comprenant l'administration d'un ou plusieurs agents chimiothérapeutiques au patient ou le traitement du patient par radiothérapie.

32. Formulation de l'analogue du peptide-2 de type glucagon (GLP-2) destinée à être utilisée dans un procédé de traitement selon la revendication 31, la formulation étant utilisée dans le traitement et/ou la prévention d'un effet secondaire d'un traitement par chimiothérapie ou radiothérapie, l'effet secondaire de la chimiothérapie étant facultativement une diarrhée, des crampes abdominales, des vomissements ou des dommages structurels et fonctionnels de l'épithélium intestinal consécutifs au traitement par chimiothérapie.

33. Formulation de l'analogue du peptide-2 de type glucagon (GLP-2) destinée à être utilisée dans un procédé de traitement selon l'une quelconque des revendications 30 à 32, le patient humain étant un patient souffrant de syndrome de l'intestin court avec défaillance intestinale.

34. Formulation de l'analogue du peptide-2 de type glucagon (GLP-2) destinée à être utilisée dans un procédé de traitement selon l'une quelconque des revendications 30 à 33, le patient humain étant un patient étant à la limite entre un patient atteint de syndrome de l'intestin court avec insuffisance intestinale et de syndrome de l'intestin court avec défaillance intestinale.

35. Formulation de l'analogue du peptide-2 de type glucagon (GLP-2) destinée à être utilisée dans un procédé de traitement selon l'une quelconque des revendications 28 à 34, le procédé comprenant l'administration de l'analogue de GLP-2 au patient une ou deux fois par jour, ou le procédé comprenant l'administration de l'analogue de GLP-2 au patient une ou deux fois par semaine.

36. Utilisation d'une formulation comprenant un analogue du peptide-2 de type glucagon (GLP-2), l'analogue de GLP-2 étant représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
dans laquelle :
R¹ est hydrogène, alkyle en C₁₋₄ (par exemple, méthyle), acétyle, formyle, benzoyle ou trifluoroacétyle X5 est Ser ou Thr
X11 est Ala ou Ser
R² est NH₂ ou OH ;
Z¹ et Z² sont indépendamment absents ou une séquence peptidique de 1 à 6 motifs d'acide aminé Lys ;
ou un sel ou dérivé pharmaceutiquement acceptable de celuici ;
pour fournir une formulation pharmaceutique liquide qui est stable pendant 18 mois lorsqu'elle est conservée à 2 à 8 °C,
la formulation comprenant :
(a) l'analogue de GLP-2 à une concentration d'environ 1 mg/ml à environ 30 mg/ml ;
(b) du méta-crésol ou du phénol en tant que conservateur à une concentration de 1,0 à 5,0 mg/ml ;
(c) un tampon choisi dans le groupe constitué d'un tampon histidine, un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ;
(d) un modificateur de tonicité non ionique choisi dans le groupe constitué du mannitol, du saccharose, du glycérol, du sorbitol et du tréhalose à une concentration d'environ 20 mM à environ 360 mM ;
(e) un pH d'environ 5,0 à environ 6,8.

37. Utilisation d'une formulation comprenant un analogue du peptide-2 de type glucagon (GLP-2), l'analogue de GLP-2 étant représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
dans laquelle :
R¹ est hydrogène, alkyle en C₁₋₄ (par exemple, méthyle), acétyle, formyle, benzoyle ou trifluoroacétyle X5 est Ser ou Thr
X11 est Ala ou Ser
R² est NH₂ ou OH ;
Z¹ et Z² sont indépendamment absents ou une séquence peptidique de 1 à 6 motifs d'acide aminé Lys ;
ou un sel ou dérivé pharmaceutiquement acceptable de celuici ;
pour fournir une formulation pharmaceutique liquide qui est stable pendant 18 mois lorsqu'elle est conservée à 2 à 8 °C,
la formulation comprenant :
(a) l'analogue de GLP-2 à une concentration d'environ 1 mg/ml à environ 30 mg/ml ;
(b) du méta-crésol ou du phénol en tant que conservateur à une concentration de 1,0 à 5,0 mg/ml ;
(c) un tampon choisi dans le groupe constitué d'un tampon histidine, un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ;
(d) un modificateur de tonicité non ionique choisi dans le groupe constitué du mannitol, du saccharose, du glycérol, du sorbitol et du tréhalose à une concentration d'environ 20 mM à environ 360 mM ;
(e) un pH d'environ 5,5 à environ 7,0 ; et
(f) du propylène glycol à une concentration de 20 mM à 300 mM.

38. Procédé de production d'une formulation pharmaceutique multidose liquide stable, la formulation comprenant un analogue du peptide-2 de type glucagon (GLP-2), dans lequel l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
dans laquelle :
R¹ est hydrogène, alkyle en C₁₋₄ (par exemple, méthyle), acétyle, formyle, benzoyle ou trifluoroacétyle X5 est Ser ou Thr
X11 est Ala ou Ser
R² est NH₂ ou OH ;
Z¹ et Z² sont indépendamment absents ou une séquence peptidique de 1 à 6 motifs d'acide aminé Lys ;
ou un sel ou dérivé pharmaceutiquement acceptable de celuici ;
le procédé comprenant la formulation (a) de l'analogue du GLP-2 à une concentration d'environ 1 mg/ml à environ 30 mg/ml ; (b) du métacrésol ou du phénol en tant que conservateur à une concentration de 1,0 à 5,0 mg/ml ; (c) un tampon choisi dans le groupe constitué d'un tampon histidine,
un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ; (d) un modificateur de tonicité non ionique choisi dans le groupe constitué du mannitol, du saccharose, du glycérol, du sorbitol et du tréhalose à une concentration d'environ 20 mM à environ 360 mM ; à un pH d'environ 5,0 à environ 6,8.

39. Procédé de production d'une formulation pharmaceutique multidose liquide stable, la formulation comprenant un analogue du peptide-2 de type glucagon (GLP-2), dans lequel l'analogue de GLP-2 est représenté par la formule :
R¹-Z¹-His-Gly-Glu-Gly-X5-Phe-Ser-Ser-Glu-Leu-X11-Thr-Ile- Leu-Asp-Ala-Leu-Ala-Ala-Arg-Asp-Phe-Ile-Ala-Trp-Leu-Ile- Ala-Thr-Lys-Ile-Thr-Asp-Z²-R²
dans laquelle :
R¹ est hydrogène, alkyle en C₁₋₄ (par exemple, méthyle), acétyle, formyle, benzoyle ou trifluoroacétyle
X5 est Ser ou Thr
X11 est Ala ou Ser
R² est NH₂ ou OH ;
Z¹ et Z² sont indépendamment absents ou une séquence peptidique de 1 à 6 motifs d'acide aminé Lys ;
ou un sel ou dérivé pharmaceutiquement acceptable de celuici ;
le procédé comprenant la formulation (a) de l'analogue du GLP-2 à une concentration d'environ 1 mg/ml à environ 30 mg/ml ; (b) du métacrésol ou du phénol en tant que conservateur à une concentration de 1,0 à 5,0 mg/ml ; (c) un tampon choisi dans le groupe constitué d'un tampon histidine, un tampon mésylate, un tampon acétate, un tampon glycine, un tampon lysine, un tampon TRIS, un tampon Bis-Tris et un tampon MOPS, le tampon étant présent à une concentration d'environ 5 mM à environ 50 mM ; (d) un modificateur de tonicité non ionique choisi dans le groupe constitué du mannitol, du saccharose, du glycérol, du sorbitol et du tréhalose à une concentration d'environ 20 mM à environ 360 mM ; à un pH d'environ 5,5 à environ 7,0.
